# EUROPEAN PATENT SPECIFICATION

(11) **EP 2 964 745 B1**
(45) Date of publication and mention of the grant of the patent: **09.07.2025**
(21) Application number: 14759572.2
(22) Date of filing: 04.03.2014
(51) Int. Cl.: A61B 1/12, A61M 39/08, A61M 39/16, A61M 39/18, C12Q 1/22, G01N 31/22

(54) **DEVICES AND METHODS FOR TESTING THE CLEANLINESS OF MEDICAL INSTRUMENTS**
VORRICHTUNGEN UND VERFAHREN ZUR PRÜFUNG DER SAUBERKEIT VON MEDIZINISCHEN INSTRUMENTEN
DISPOSITIFS ET PROCÉDÉS PERMETTANT DE TESTER LA PROPRETÉ D'INSTRUMENTS MÉDICAUX

(30) Priority: 06.03.2013 US 201361773419 P
(43) Date of publication of application: 13.01.2016
(73) Proprietor: RUHOF CORPORATION, Mineola NY 11501-1919 (US)
(72) Inventor: MACKAY, Douglas, Mineola, NY 11501-1919 (US); ESQUENET, Marc, B., Mineola, NY 11501-1919 (US); ESQUENET, Bernard, E., Mineola, NY 11501-1919 (US); RUVINSKY, Lee, A., Mineola, NY 11501-1919 (US)
(74) Representative: Finnegan Europe LLP
(86) International application number: PCT/US2014/020267
(87) International publication number: WO 2014/138043

(56) References cited:
- EP-A1- 2 397 066
- WO-A2-2008/048823
- DE-A1- 102011 050 765
- DE-A1- 102011 050 765
- US-A1- 2006 102 200
- US-A1- 2006 102 200
- US-A1- 2008 027 379
- US-A1- 2009 043 372
- US-A1- 2010 136 670
- US-A1- 2011 289 705
- US-A1- 2012 100 531

## Description

### TECHNICAL FIELD

The present disclosure is directed towards methods for testing the cleanliness of medical instruments.

### BACKGROUND

Endoscopes and other elongated medical instruments are relatively expensive products because they are typically configured for multiple uses. These instruments include one or more cannulas or lumens, through which surgical implements and other devices are passed during a surgical procedure. During use, a cannula may be exposed to bodily fluids and other materials that can accumulate on an inner surface of the lumen. If the accumulated material is not thoroughly cleaned from the inner surface prior to disinfection and sterilization, surgical debris can be passed to another patient, leading to infection or other complications. It is thus critical to properly clean the interior surfaces of endoscopes and similar surgical instruments. However, these medical devices can include long, narrow, tortuous lumens, which are often difficult to access and clean properly.

Another challenge includes properly determining when a cannulated device has been properly cleaned. Lumens of such device are difficult or impossible to inspect visually. Standard cleaning protocols can be followed but these do not always result in a properly cleaned device. Consequently, improved testing methods and devices are required to ensure an elongated medical instrument is properly cleaned.

Various techniques and devices have been previously proposed for cleaning cannulated instruments. The simplest procedure involves immersing the devices in solutions containing a detergent. Other applications use a small flexible brush, much like the conventional bottle brush having bristles locked between twisted wires. Such brushes are not entirely effective as they do not always evenly contact all the inner surfaces of a lumen. In addition, the bristles can scratch or damage the interior surfaces of the endoscopes and leave hardened deposits behind.

One possible solution to this problem is disclosed by U.S. Patent Application Publication No. US 2003/0213501A1, which describes a hydrophilic polyurethane coating deposited on the bristles of a conventional endoscopic cleaning brush. Another solution is disclosed by U.S. Patent No. 6,699,331, which describes a wiping member configured to uniformly distribute the contaminates on the internal wall of the lumen and treating the resulting film with an enzyme. Yet another solution is disclosed by U.S. Patent No. 6,045,623, which describes both a brush and a swab attached to a shaft for cleaning lumens. Another cleaning system uses a polyurethane foam immersed in an enzymatic cleaning solution, as described in commonly assigned U.S. Patent Application Publication No. 2006/0102200. However, none of these devices always provide perfect cleaning of medical instructions, necessitating testing cleaning effectiveness.

One method for testing the cleanliness of an instrument following a cleaning protocol includes passing a test swab through the "cleaned" cannula and analyzing the test swab for any biological debris or bioburden. One test of bioburden relies on the presence of adenosine triphosphate (ATP). ATP is a molecule found in and around living cells, and can provide a direct measure of biological concentration. However, current systems and methods cannot test for ATP where biological cells are contained within a biofilm.

A biofilm is produced by a complex and coordinated network of microbes having increased resistance to detergents and antibiotics. Microbes within the network form an organic polymer matrix, producing a sticky mucous coating, or slime. The matrix provides structural support for cellular communities formed within the network. Channels may distribute nutrients within the network, allowing the communities to grow in a more isolated environment.

Biofilms can include a variety of microbes, including aerobic and anaerobic bacteria, algae, protozoa, and fungi. The bacteria in a biofilm can have significantly different properties from free-floating bacteria due to the complex matrix structure. For example, microbial cells within the matrix may have unique gene expression. This may allow synergistic interactions within the complex network.

DE102011050765 discloses a method for determining degree of pollution of a narrow human medical device. The method involves inserting or removing a clean swab of a wire into a cavity of a medical device. The clean swab is introduced into a transparent test vessel using a holder fixed at an end of the wire for detection of adenosine triphosphate (ATP) or adenosine monophosphate (AMP). The clean swab is left in the test container for predetermined time. The test container is introduced into the measuring device for luminescence measurement.

Current devices and methods are not able to consistently extract ATP from biological cells contained within a protective biofilm. Consequently, current devices and methods may underreport the true level of cleanliness of a medical instrument if the testing relies upon ATP analysis. The present disclosure provides improved devices and methods for testing the cleanliness of cannulated medical instruments.

### SUMMARY

One aspect of the present invention provides a method testing a cleanliness of a cannula of a medical instrument using a testing device, wherein the testing device comprises a guiding member having a rounded first end and a second end releasably coupled to a sponge element containing a dried extractant. The method steps include wetting the sponge element in at least one of sterile water and ATP-free water to activate the dried extractant and inserting the first end of the guiding member into a proximal end of the cannula. The guiding member is then pushed into the cannula to pass the first end of the guiding member through the cannula until the first end of the guiding member exits a distal end of the cannula. The guiding member is then pulled out of the distal end of the cannula and an inner surface of the cannula is contacted with the activated extractant to lyse a cell located on the inner surface of the cannula; pulling the second end of the guiding member and the sponge element out of the distal end of the cannula; detaching the sponge element from the guiding member; introducing the sponge element into a test swab tube; and exposing the sponge element to a reagent in the test swab tube.

Further aspects of the present disclosure are the subject of the dependent claims.

Additional objects and advantages of the present disclosure will be set forth in part in the description which follows, and in part will be obvious from the description, or may be learned by practice of the present disclosure.

It is to be understood that both the foregoing general description and the following detailed description are exemplary and explanatory only and are not restrictive of the present disclosure, as claimed.

The accompanying drawings, which are incorporated in and constitute a part of this specification, illustrate several embodiments of the present disclosure and together with the description, serve to explain the principles of the present disclosure.

### BRIEF DESCRIPTION OF THE DRAWINGS

FIG. 1 is a schematic diagram of a device for cleaning a cannulated medical instrument, according to an exemplary embodiment.
FIG. 2 is an enlarged view of a portion of a device for cleaning a cannulated medical instrument, according to an exemplary embodiment.
FIG. 3A, 3B, 3C, and 3D are side views of different configurations of a foam element, according to various exemplary embodiments.
FIG. 4 is an enlarged view of a bristle element, according to an exemplary embodiment.
FIG. 5A is a schematic diagram of a device for cleaning a cannulated medical instrument, according to an alternate exemplary embodiment.
FIG. 5B is an enlarged view of a portion of a device for cleaning a cannulated medical instrument, according to an alternate exemplary embodiment.
FIG. 5C is a schematic diagram of a device for cleaning a cannulated medical instrument, according to an alternate exemplary embodiment.
FIG. 6 is a flow diagram illustrating a method of using a device for cleaning a cannula of a medical instrument, according to an exemplary embodiment.
FIG. 7A is a schematic diagram of a testing device for testing the cleanliness of a cannulated medical instrument, according to an exemplary embodiment.
FIG. 7B is an enlarged view of a portion of a testing device for testing the cleanliness of a cannulated medical instrument, according to an exemplary embodiment.
FIG. 7C is a schematic diagram of a testing device for testing the cleanliness of a cannulated medical instrument, according to an exemplary embodiment.
FIG. 8 is a flow diagram illustrating a method of using a testing device for testing the cleanliness of a cannulated medical instrument, according to an exemplary embodiment.
FIG. 9 is a schematic diagram of a system for cleaning and testing the cleanliness of a cannulated medical instrument, according to an exemplary embodiment.
FIG. 10 is a schematic diagram of a kit for testing the cleanliness of a cannulated medical instrument, according to an exemplary embodiment.

Reference will now be made in detail to the present embodiments of the present disclosure, examples of which are illustrated in the accompanying drawings. Wherever possible, the same reference numbers will be used throughout the drawings to refer to the same or like parts.

### DETAILED DESCRIPTION

The present disclosure is described herein with reference to an illustrative embodiment for a particular application, such as, for example, testing the cleaning of a medical instrument. It is understood that the embodiments described herein are not limited thereto. Those having ordinary skill in the art and access to the teachings provided herein will recognize additional modifications, applications, embodiments, and substitution of equivalents that all fall with the scope of the present disclosure. Accordingly, the present disclosure is not limited by the foregoing or following descriptions.

### CLEANING DEVICE

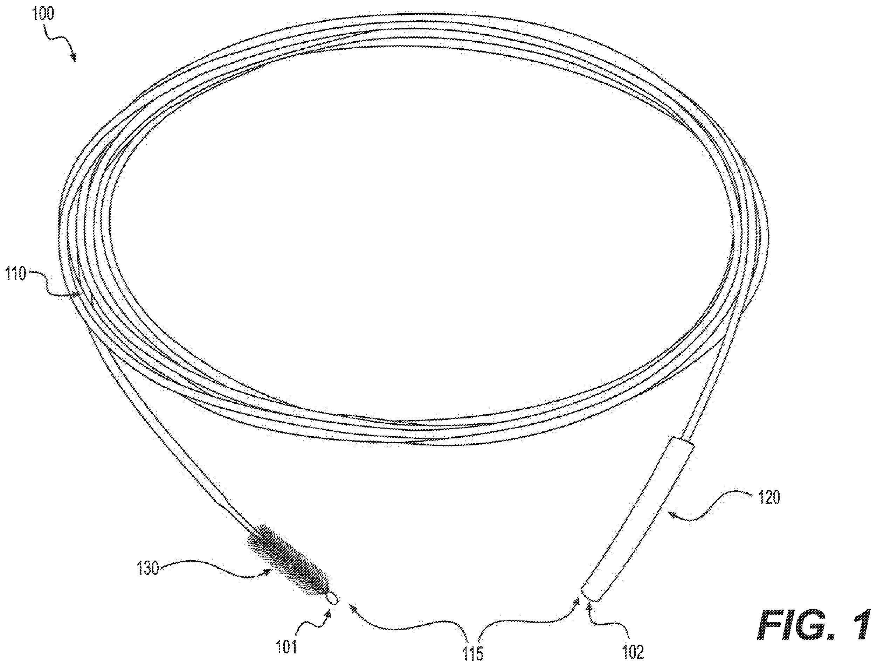

FIG. 1 shows a schematic diagram of a device 100 for cleaning a cannula of a medical instrument, according to an exemplary embodiment. The medical instrument can be a flexible or rigid scope, for example, an endoscope, a flexible fiberoptic, a laparoscopic instrument, a tracheostomy tube, a proctoscope, a bronchoscope, orthopedic instruments, part of an instrument or any medical device with a cannula or a lumen. The medical instrument can be articulated or non-articulated. A cannula of a medical instrument can vary in length and shape. For example, the length of a cannula can range from about 50 mm to about 500 mm. While the cross-sectional geometry of a cannula can vary, they are generally circular, oval, or have an arcuate shape.

Device 100 can comprise an elongated flexible guiding member 110 having a first end 101 and a second end 102. Guiding member 110 can be flexible along its longitudinal length and configured to follow and flex based on the geometry of a cannula of the medical instrument being cleaned. Guiding member 110 can be made of flexible polypropylene, polyoxmethylene, or other flexible polymers or plastics. The diameter of guiding member 110 can range between 0.5 mm to 5 mm.

Device 100 can further comprise a plurality of cleaning elements 115 attached to guiding member 110. For example, plurality of cleaning elements 115 can be located between first end 101 and second end 102. In some embodiments, plurality of cleaning elements 15 can comprise a foam element 120 and a bristle element 130. In other embodiments, plurality of cleaning elements 115 can be located at one end of guiding member 110 or at some other locations along guiding member 110.

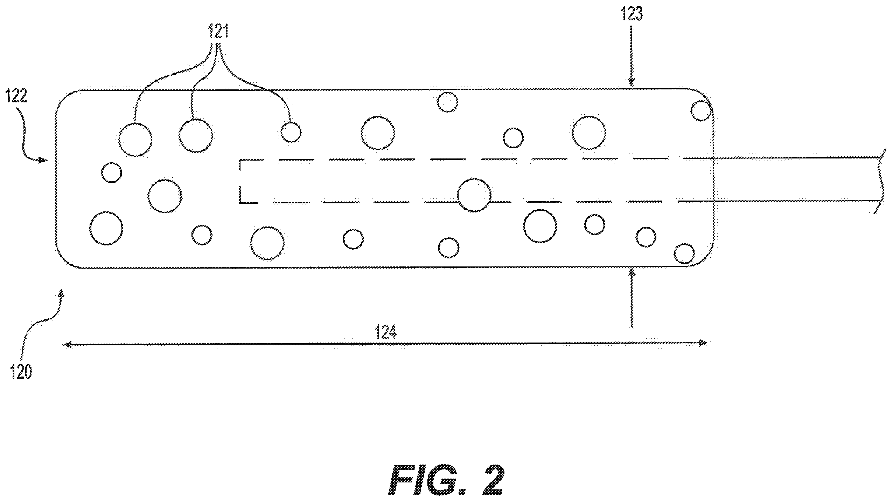

As shown in FIG. 2, foam element 120 can comprise a foam structure 122. Foam structure 122 can comprise a hydrophobic medical grade polyurethane foam that forms a backbone for an open cell foam. A coating of hydrophilic medical grade polyurethane can be formed on the hydrophobic backbone. Foam structure 122 can have a high porosity resulting in a plurality of pores 121. Plurality of pores 121 can vary in size and shape.

Foam structure 122 can be generally cylindrical or another suitable shape. The cross-sectional diameter of foam element 120 can be approximately equal to or greater than the inner diameter of the cannula being cleaned. A cross-sectional diameter 123 of foam element 120 can be between about 1 mm to about 14 mm. Cross-sectional diameter 123 of foam element 120 can be selected based on the dimension of the cannula being cleaned. For example, cross-sectional diameter 123 can be greater than the cross-sectional diameter of the cannula being cleaned, but not so much greater that foam element 120 is unable to compress and fit within the cannula. A length 124 of foam element 120 can range between about 0.25 to about 5 inches.

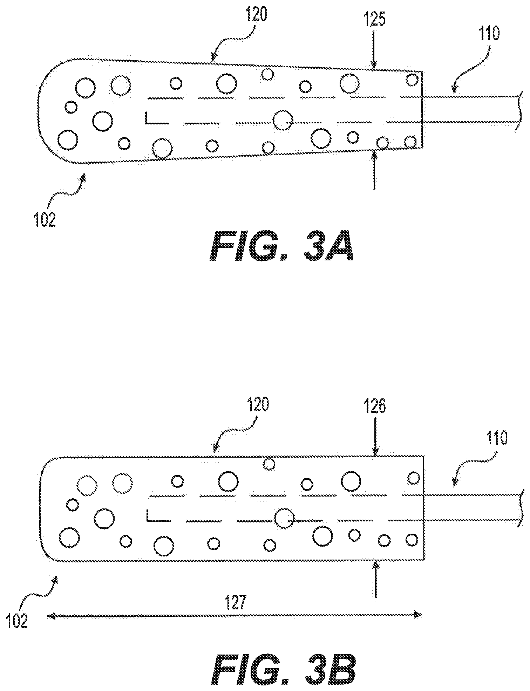

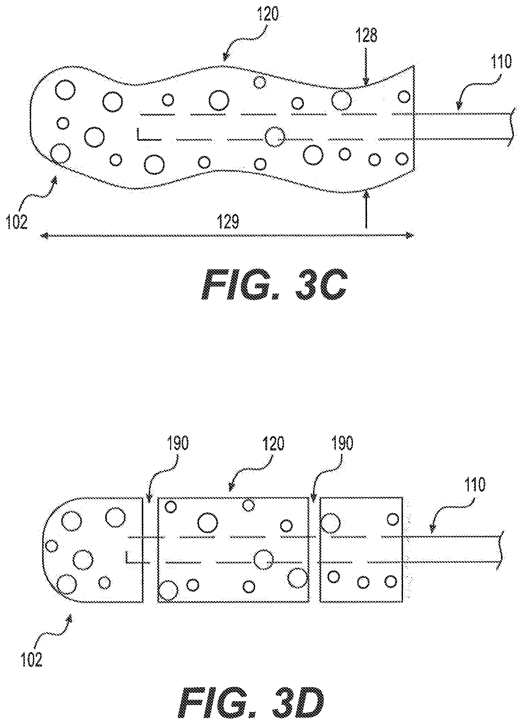

In various other embodiments, the cross-sectional diameter of foam element 120 can vary along length 124 of foam element 120. For example, in FIG. 3A, a cross-sectional diameter 125 decreases moving away from second end 102. In contrast, in FIG. 3B a cross-sectional diameter 126 remains substantially constant over a length 127 of foam element 120. In FIG. 3C, a cross-sectional diameter 128 varies along a length 129 of foam element 120 creating a wave like element. Additional embodiments of foam element 120 are contemplated. For example, FIG. 3D illustrates an additional embodiment of foam element 120 wherein foam element 120 includes multiple foam sections spaced apart creating a plurality of cavities 190 between each section. Cavities 190 can act as collection chambers for bioburden, reducing the amount of bioburden collected on the exterior surface of foam element 120. In other embodiments, it is contemplated that surface features (e.g., helical slots, protrusions, dimples, ridges, etc.) can be used to enhance bioburden removal.

Foam element 120 can be attached to guiding member 110 using an adhesive material, for example, an epoxy or glue, as would be apparent to those of ordinary skill in the art. In another embodiment, foam element 120 can be attached to guiding member 110 using a heat bonding method that can eliminate the need for an adhesive material. The adhesive or heat bonding method used can be configured to ensure that foam element 120 will not disconnect from guiding member 110 during use.

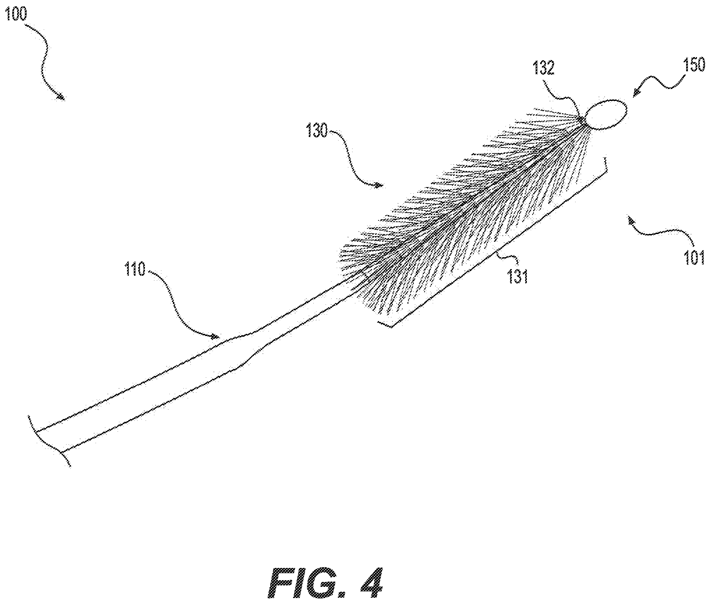

FIG. 4 illustrates an enlarged view of bristle element 130. Bristle element 130 can be located in the region of first end 101. Bristle element 130 can be comprised of a plurality of bristles 131 secured between a plurality of twisted metal wires 132 protruding from guiding member 110. Bristles 131 can be made of nylon. It is contemplated that other methods apparent to those of ordinary skill in the art for securing bristles to a brush or a support member are contemplated.

Bristles 131 can extend out laterally from twisted metal wires 132 forming a generally cylindrical shape. Bristles 131 in addition to extending out laterally can be angled toward first end 101 or away from first end 101. The diameter of the generally cylindrical shape bristle element 130 can be about 1 mm to about 14 mm. The cross-sectional diameter of bristle element 130 can correspond to the cross-sectional diameter of foam element 120 and can be selected based on the dimension of the cannula being cleaned. It is contemplated that the cross-sectional diameter of bristle element 130 can be less than, equal to, or greater than the diameter of the foam element 120. The length of bristle element 130 can range between about 0.25 to about 3 inches. In various embodiments, varying the length of bristles 131 can create ridges or a helical pattern along the exterior of bristle element 130.

Device 100 can further comprise a bulb 150 at first end 101 of device 100. Bulb 150 can include a rounded protrusion covering the end of metal wires 132. Bulb 150 can be made of rubber, plastic or polymer, for example, polypropylene. In operation, bulb 150 can be configured to slide freely over edges and rough surfaces to allow for easy insertion and guiding through the cannula being cleaned.

In various other embodiments (not shown), device 100 can include additional cleaning elements beyond just foam element 120 and bristle element 130 described above. For example, device 100 can comprise a plurality of bristle elements 130 extending along guiding member 110 spaced at different intervals. Alternatively, device 100 can comprise a plurality of foam elements 120 extending along guiding member 110 spaced at different intervals. In yet another embodiment, device 100 can comprise a plurality of both foam elements 120 and bristle elements 130.

In addition to varying the number of cleaning elements, the location of the cleaning elements can also vary. For example, foam element 120 can be located in the region of first end 101 and bristle element 130 can be located in the region of second end 102. In yet another embodiment, both foam element 120 and bristle element 130 can be located in the region of either first end 101 or second end 102.

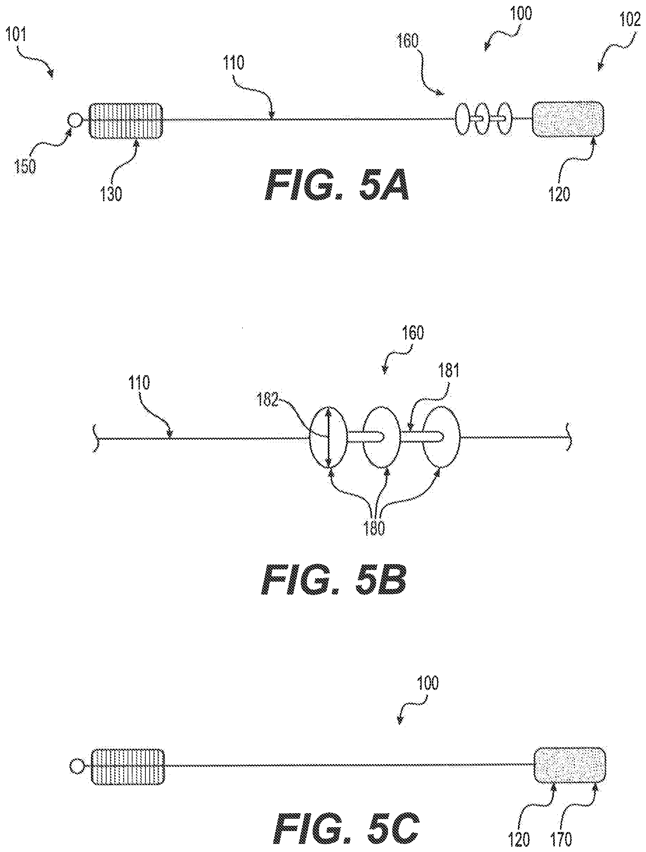

Device 100 can also include one or more different types of cleaning elements. For example, as shown in FIG. 5A, device 100 can include a squeegee element 160 in addition to foam element 120 and bristle element 130. As shown in FIG. 5B, squeegee 160 can comprise at least one of a wiping member 180 attached to guiding member 110 located between first end 101 and second end 102. Two adjacent wiping members 180 can be spaced apart while coupled by a separating member 181.

It is also contemplated that device 100 could include only foam element 120 and squeegee element 160, and not bristle element 130. Other various embodiments of device 100 could include various other numbers or combinations of various types of cleaning elements.

The cross-sectional shape of wiping members 180 can be configured to correspond to the shape of a cannula being cleaned. A cross-sectional diameter 182 of wiping members 180 can be equal to or less than the diameter of a cannula being cleaned. In other embodiments, diameter 182 for each wiping member can be different, such that the diameter 182 decreases moving toward first end 101. Wiping members 180 can be configured to contact the inner surface of the cannula being cleaned and sweep material dislodged by bristle element 130. Squeegee element 160 and wiping members 180 can be flexible or rigid and formed of polyurethane, polypropylene, or other polymer or plastic.

Device 100 as described above with regard to the various embodiments can be characterized as a mechanical cleaning device. However, in order to enhance the cleaning effectiveness of device 100 the mechanical cleaning can be combined with chemical cleaning. According to an exemplary embodiment, one or more of cleaning elements 115 can be combined with a cleaning solution 170. For example, cleaning solution 170 can be an enzymatic cleaner of the type configured to degrade, disperse, or dissolve biological contaminant. These can include ENDOZIME^{®} AW Triple Plus^{®} with A.P.A. or ENDOZIME^{®} Bio-Clean (Ruhof Corp., NY). Other enzymatic cleaners configured to disperse and dissolve biological contaminant (e.g., bioburden) can be used. Cleaning solution 170 can take the form of a liquid, gel, foam, solid, paste, or spray cleaner and may be applied to foam element 120.

According to various embodiments, as shown in FIG. 5C, device 100 can comprise foam element 120 containing cleaning solution 170. In one embodiment, foam element 120 can be immersed in cleaning solution 170 and used immediately for cleaning a medical instrument. In an alternate embodiment, foam element 120 can be immersed in cleaning solution 170 and then passed through a dryer, of known construction, wherein excess water is removed without destruction of the enzymes. For example, foam element 120 can be dipped in cleaning solution 170 for between about 1 second and 5 seconds and then removed to dry. Following drying, device 100 can be packaged or stored until shipment to the end user. Device 100 can be individually packaged in an ATP-free environment or sterile packaged. Before use, device 100 can be removed from its package and foam element 120 immersed in sterile or ATP-free water to activate cleaning solution 170.

### CLEANING METHOD

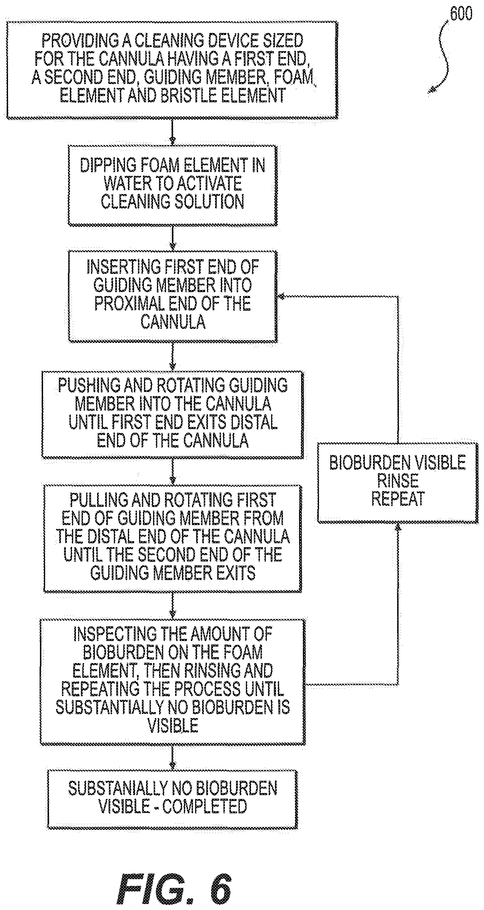

Device 100 as described above can be utilized as an instrument for cleaning a cannula of a medical instrument. FIG. 6 shows a flow chart 600 illustrating the method of cleaning a cannula of a medical instrument using device 100.

Device 100 can be provided in a package (e.g., single use package) that identifies the dimensions of foam element 120 and bristle element 130 in order to allow the end user to select the correct device 100 size based on a cannula being cleaned. In an alternate embodiment, instead of the package identifying the dimensions of foam element 120 and bristle element 130, the package can identify the dimensions of the target cannula.

In yet another embodiment, guiding member 110 can be color coded based on the dimension of cleaning elements 115. For example, yellow can indicate foam element 120 has about a 2 mm cross-sectional diameter, red can indicate foam element 120 has about a 3 mm cross-sectional diameter, and purple can indicate foal element 120 has about a 4 mm cross-sectional diameter. It is contemplated that additional colors and diameters can be used.

After the user removes device 100 from the package, the user can immerse foam element 120, which can be combined with cleaning solution 170, in clean water to reactivate cleaning solution 170. In an alternate embodiment, as described above, foam element 120 can be immersed in cleaning solution 170 immediately prior to use.

Next, the user can insert first end 101 into the proximal end of a cannula of a medical instrument. The user can then feed guiding member 110 through the cannula. If there is some resistance from bioburden lodged in the cannula, the user can move device 100 back and forth until the path is clear. The user can then continue feeding device 100 through the cannula.

The user can continue to feed guiding member 110 until bulb 150 exits from the distal end of the cannula. Once bulb 150 exits from the distal end, the user can reorient the medical instrument being cleaned and begin pulling on device 100, drawing device 100 through the cannula from the distal end instead of pushing it through from the proximal end.

Bristle element 130 moving through the cannula first can dislodge and break up large pieces of bioburden and carry out many of the pieces. Subsequently, foam element 120 can carry out the remainder of the pieces using the flat face of foam element 120 as a plowing surface. In addition, foam element 120 can also contact the interior surface of the cannula and mechanically and chemically cleaning the surface. The user can continue to pull device 100 until foam element 120 exits the distal end of the cannula. It can be advantageous to not pull device 100 back through the proximal end because bioburden may be redeposited within the cannula. In addition, the user can rotate guiding member 110 while pushing and pulling in order to cause rotation within the cannula to enhance cleaning.

Once device 100 is completely removed from the cannula, device 100 can be thoroughly rinsed to remove all signs of bioburden. Particularly, foam element 120 and bristle element 130 can be thoroughly rinsed.

The user can then repeat the above process one or more times until there is substantially no visible debris left on foam element 120 following removal from the cannula.

Device 100 can be configured for single use. Once a cannula is cleaned, device 100 can be disposed of according to proper procedure. In alternate embodiments, device 100 can be cleaned, sanitized and stored for later reuse.

### TESTING DEVICE

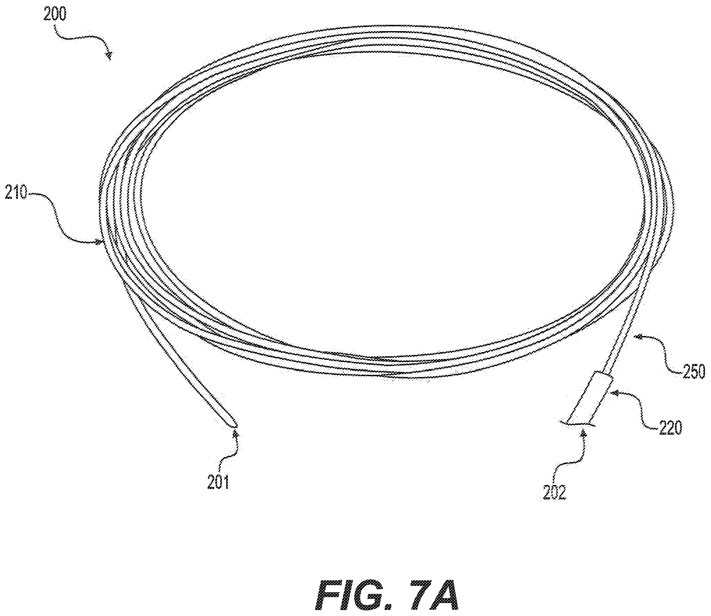

Following a pre-cleaning, partial cleaning, or allegedly complete cleaning of the medical instrument or a portion of the medical instrument, a test can be performed to determine the cleanliness of one or more cannulas or lumens within the medical instrument. Accordingly, FIG. 7A shows a schematic diagram of a testing device 200 configured for testing the cleanliness of a cannula or lumen of a medical instrument as described above. Testing device 200 can include one or more features of device 100 previously described. For example, testing device 200 could include one or more of foam element 120, bristle element 130, squeegee element 160, or other type of cleaning element.

Testing device 200 can comprise an elongated flexible guiding member 210 having a first end 201 and a second end 202. Guiding member 210 can be flexible along its longitudinal length and configured to follow and flex based on the geometry of a cannula of the medical instrument being tested. Guiding member 210 can be made of flexible polypropylene, polyoxmethylene, or other flexible polymers or plastics. The diameter of guiding member 110 can range between 0.5 mm to 5 mm.

Testing device 200 can further comprise a sponge element 220 attached to coupling member 250. Sponge element 220 can be attached using an adhesive material, for example, an epoxy or glue, as would be apparent to those of ordinary skill in the art. In another embodiment, sponge element 220 can be attached using a heat bonding method to eliminate the need for an adhesive material. The adhesive or heat bonding method used can be configured to ensure that sponge element 220 will not disconnect from coupling member 250 during use, avoiding dislodgment within the cannula during testing. Sponge element 220 can include one or more features of foam element 120 described above.

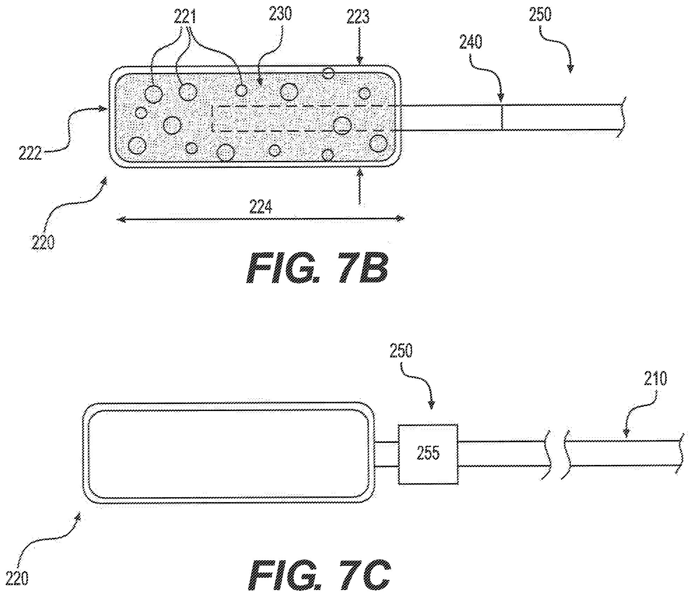

As shown in FIG. 7B, sponge element 220 can comprise a foam structure 222. Foam structure 222 can comprise a hydrophobic medical grade polyurethane foam that forms a backbone for an open cell foam. A coating of hydrophilic medical grade polyurethane can be formed on the hydrophobic backbone. Hydrophilic polyurethanes are water-loving and absorb liquids to a greater degree than hydrophobic polyurethane. However, the physical strength and tensile strength of hydrophilic materials is less than that of hydrophobic materials. Therefore, the composite material used as the foam structure 222 provides benefits of both materials. Foam structure 222 can have a high porosity resulting in a plurality of pores 221. Plurality of pores 221 can vary in size and shape.

Foam structure 222 can be generally cylindrical or another suitable shape. A cross-sectional diameter 223 of sponge element 220 can be between about 1 mm to about 14 mm. Cross-sectional diameter 223 of sponge element 220 can be approximately equal to or greater than the inner dimension of the cannula being cleaned. Cross-sectional diameter 223 of sponge element 220 can be selected based on the dimension of the cannula being tested. For example, cross-sectional diameter 223 can be greater than the cross-sectional diameter of the cannula being cleaned, but not so much greater that sponge element 220 is unable to compress and fit within the cannula. A length 224 of sponge element 220 can range between about 0.25 inches to about 2 inches. As described above for foam element 120, sponge element 220 can be provided in various configurations.

As shown in FIG. 7B, sponge element 220 can be impregnated with an extractant 230. Sponge element 220 can either be immersed in extractant 230 immediately prior to use or extractant 230 can be dried onto sponge element 220 and activated immediately prior to use. In some embodiments, sponge element 220 can be configured to absorb and retain water to activate extractant 230 in less than about 5 min. In some embodiments, sterile water or ATP-free water can be used to activate extractant 230.

Extractant 230 can comprise a detergent-based preservative and extractant configured to open biological cells and release ATP. For example, intracellular ATP can be released to enhance detection of biological cells contained within a biofilm. For example, extractant 230 can comprise Triton X-100, a quaternary-based detergent, tricholoacetic acid, and protocatechuic acid. In addition, extractant 230 can further comprise a buffering solution. The immersion of sponge element 220 in extractant 230 can also act as a pretreatment of sponge element 220, removing latent ATP.

Traditionally, swabbing a cannula collects mostly extracellular ATP because intracellular ATP remaining within cells in a biofilm may not be detected. By not lysing biological cells within the biofilm, prior devices for testing cleanliness using ATP analysis may have provided inaccurate results. Collecting and testing for only the extracellular ATP is not always accurate because these protocols fail to detect biological cells present in the biofilm. To mitigate these problems with prior art devices and methods, the present disclosure provides extractant 230 to lyse the biofilm's cells. Releasing intracellular ATP allows for its collection and subsequent analysis using the devices and methods described herein.

In an alternate embodiment, as described above for foam element 120, testing device 200 can comprise a plurality of sponge elements 220 or other cleaning elements. A sponge element located nearest first end 201 can contain extractant 230, while other sponge elements may not contain extractant. Such a configuration of sponge elements can provide more time for extractant 230 to react with any bioburden present in the cannula before the other sponge elements pass over the bioburden and collect it. Various other configurations of elements associated with testing device 200 are also contemplated, where some may include extractant 230 and some may not.

Coupling member 250 can be configured to uncouple sponge element 220 from guiding member 210 following the swabbing of a cannula. Coupling member 250 can include a reduced cross-sectional area configured to preferentially detach. Coupling member 250 could also include a region configured for detachment using another device. For example, coupling member 250 could be configured to be cut, snapped, or severed using scissors, blade, forceps, or other similar device.

To facilitate uncoupling, coupling member 250 can further comprise a crease 240. Crease 240 can be located be located less than about 2.5 inches from sponge element 220. Crease 240 can be configured to snap with repeated bending. Alternatively, crease 240 can also be configured to indicate the location where coupling member 250 should be cut to provide a suitable size for subsequent analysis. In other embodiments, crease 240 can take the form of an indicia, perforated edge, indentation, reduced cross-sectional area, protrusion, or similar structure. Coupling member 250 could be color coded to distinguish guiding member 210.

As shown in FIG. 7C, coupling member 250 can include a release mechanism 255. Release mechanism 255 can include a latch, clip, hook, loop, or other type of detachment mechanism. In some embodiments, coupling member 250 or element 255 can be flexible to pass through a cannula. As such, release mechanism can have an outer dimension of less than about 2 mm.

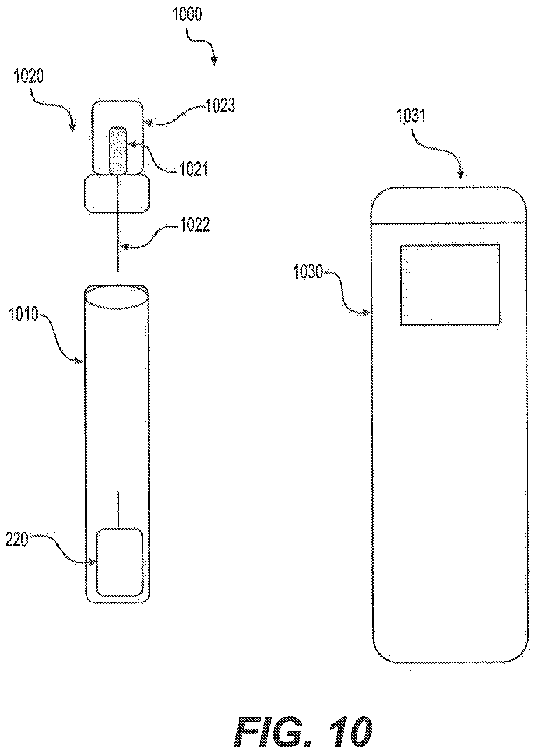

Sponge element 220 can be analyzed using various devices or systems. Uncoupling sponge element 220 from guiding member 210 can facilitate analysis of sponge element 220. For example, sponge element 220 can be analyzed for ATP using the ATP Complete^{®} hand held device test kit (Ruhof Corp., NY). According to an exemplary embodiment, FIG. 10, shows parts of a device test kit 1000. Device test kit 1000 can comprise a test swab tube 1010, a test swab 1020, and a testing device 1030. Testing device 1030 can be hand-held.

Test swab tube 1010 can be a generally cylindrical container sealed at the bottom with an opening at the top. Test swab tube 1010 can be configured to receive sponge element 220.

Test swab 1020 can be a cap container configured to be releasably coupled to test swab tube 1010. Test swab 1020 can include a reagent 1021 within an upper portion 1023 of test swab 1020. Reagent 1021 can include a solution containing liquid-stable luciferase/luciferin. Other types of solid, liquid, or gaseous forms of reagent 1021 could be used to detect ATP or another type of bioburden indicator.

Test swab 1021 can be configured to be snapped when coupled to test swab tube 1010 causing reagent 1021 to release into test swab tube 1010. Test swab 1020 can further comprise a swabbing member 1022 configured to swab a surface or provide force to maintain an item (such as an uncoupled sponge element) within test swab tube 1010.

Device 1030 can include a cover 1031 configured to open and expose an aperture configured to receive test swab tube 1010. Device 1030 can be configured to analyze the amount of contamination with the test swab tube 1010. For example, device 1030 can detect the amount of ATP collected by sponge element 220 and output a numerical value. A higher numerical value can indicate a higher level of contamination.

In other embodiments, it is contemplated that sponge element 220 may be used in conjunction with other measurement devices. For example, sponge element 220 can be used with a protein or a ninydrin measurement device.

### TESTING METHOD

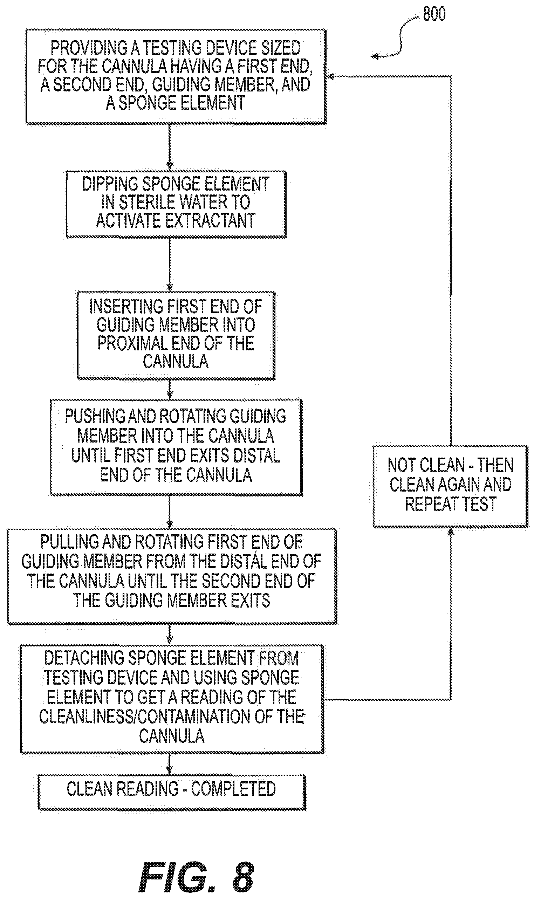

Testing device 200 as described above can be utilized as an instrument for testing the cleanliness of a cannula of a medical instrument. FIG. 8 shows a flow chart 800 illustrating the method of testing the cleanliness of the cannula of a medical instrument using testing device 200, according to an exemplary embodiment. Similar to device 100, testing device 200 can be provided in a package (e.g., single use package) that identifies the dimensions of sponge element 220 or cannula in order to allow the user to select the correct size of testing device 200, based on the size of the cannula to be cleaned.

After the user removes testing device 200 from the package, the user can immerse sponge element 220 (containing extractant 230) in sterile or ATP-free water to activate extractant 230. In an alternate embodiment, as described above, sponge element 220 can be immersed in extractant 230 immediately prior to use.

Next the user can insert first end 201 of guiding member 210 into the proximal end of the cannula of the medical instrument being tested. The user can then push guiding member 210 through the cannula until first end 201 exits the distal end of the cannula. The medical instrument can be repositioned and guiding member 210 pulled through the cannula. Pulling can continue until sponge element 220 approaches the proximal end of the cannula. Once sponge element 220 approaches the proximal end, the user can carefully pull on guiding member 210 to ensure sponge element 220 is properly inserted into the cannula.

Once sponge element 220 is in the cannula, the user can continue to pull on guiding member 210. As a result, sponge element 220 can be pulled through the cannula causing foam structure 222 immersed in extractant 230 to contact an inner surface of the cannula. Extractant 230 can cause living biological cells in the cannula to open and release intracellular ATP. Sponge element 220 can function to collect at least intracellular ATP, along with any additional bioburden present. Testing device 200 and sponge element 220 can also collect protein and ninhydrin for analysis.

The user can pull on guiding member 210 at a steady speed. Pulling on guiding member too rapidly can reduce the time extractant 230 has to react with the biological cells. As a result, the amount of intracellular ATP collected may be reduced. Therefore, the user can pull at a speed of between about 1 and about 5 inches/second.

The user can continue to pull guiding member 210 until sponge element 220 exits the distal end of the cannula. Once testing device 200 is completely removed from the cannula, sponge element 220 can be uncoupled from guiding member 210. Sponge element 220 can be uncoupled from guiding member 210 using coupling member 250. For example, coupling member 250 can be cut or crease 240 bent back and forth until it snaps.

Uncoupled sponge element 220 can be placed in test swab tube 1010, as shown in FIG. 10. The user can then place test swab 1020 on top of test swab tube 1010 and couple the two together. Next, the user can snap test swab 1020 causing reagent 1021 to flow down into test swab tube 1010. Squeezing upper portion 1023 can expedite the release of reagent 1021. The user can then gently shake test swab 1020 and test swab tube 1010 for about 1 to about 5 seconds.

Care should be taken to avoid contacting sponge element 220 with any surface that may contain ATP. For example, sponge element 220 may be placed within test swab tube 1010 and then uncoupled from guide member 210. Alternatively, sponge element 220 can be detached from guide member 210 and then placed within test swab tube 1010 if sufficient care is taken to ensure the uncoupled sponge element is not exposed to an additional source of ATP.

Following shaking, the user can open cover 1031 and insert test swab 1020 and test swab tube 1010 into the opening in device 1030. After insertion, the user can close the cover and initiate the measurement reading.

Device 1030 can be configured to analyze test swab tube 1010 to produce a signal associated with a level of cleanliness of the cannula. For example, device 1030 can output a numerical value representative of the amount of ATP. The amount of ATP can include extracellular ATP, intracellular ATP, combinations of both types of ATP, or other sources of ATP contained within a biofilm.

A numerical value associated with a representation of the signal can be provided in relative light units (RLU). Depending on numerical value, additional cleaning can be recommended. For example, a RLU reading of 0 to about 45 can indicate low contamination level and therefore additional cleaning may not be recommended. In contrast, a RLU reading of about 46 or higher can indicate a high contamination level and therefore additional cleaning may be recommended.

In various embodiments, detection devices can be utilized that detect the amount of protein, ninhydrin, intracellular ATP, combinations of these or other indicators of bioburden.

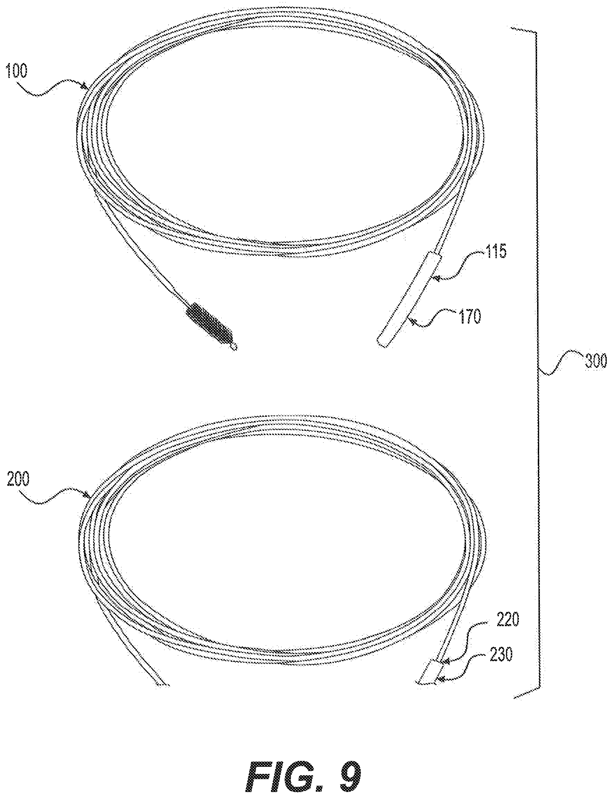

As shown in FIG. 9, device 100 and testing device 200 can be combined to form a kit 300 for cleaning and testing the cleanliness of the cannula of a medical instrument. Kit 300 can comprise at least one device 100 having a cleaning element 115 containing a cleaning solution 170 and at least one testing device 200 having a sponge element 220 containing an extractant 230. The size of both devices can correspond to the same size cannula. In addition, kit 300 can comprise additional aliquots of cleaning solution 170 and extractant 230. In other embodiments, cleaning solution 170 and extractant 230 can be provided in separate containers as a kit that can be configured to be used with a variety of cleaning and testing devices for cannulated medical instruments.

In other embodiments, one or more different types of kits 300 can be provided. For example, kit 300 could include testing device 200 and test swab tube 1010. In other embodiments, kit 300 could include test swab 1020, reagent 1021, testing device 1030, or other various combinations of the components described above.

Other embodiments of the present disclosure will be apparent to those skilled in the art from consideration of the specification and practice of the present disclosure disclosed herein. It is intended that the specification and examples be considered as exemplary only, with a true scope of the present disclosure being indicated by the following claims.

## Claims

1. A method of testing a cleanliness of a cannula of a medical instrument using a testing device (100), wherein the testing device (100) comprises a guiding member (110) having a rounded first end (101) and a second end (102) releasably coupled to a sponge element containing a dried extractant, the steps comprising:
wetting the sponge element in at least one of sterile water and ATP-free water to activate the dried extractant;
inserting the first end (101) of the guiding member (110) into a proximal end of the cannula;
pushing the guiding member (110) into the cannula to pass the first end (101) of the guiding member (110) through the cannula until the first end (101) of the guiding member (110) exits a distal end of the cannula;
pulling at least part of the guiding member (110) out of the distal end of the cannula;
contacting an inner surface of the cannula with the activated extractant to lyse a cell located on the inner surface of the cannula;
pulling the second end (102) of the guiding member (110) and the sponge element out of the distal end of the cannula;
detaching the sponge element from the guiding member (110);
introducing the sponge element into a test swab tube; and
exposing the sponge element to a reagent in the test swab tube.

2. The method of claim 1, wherein the reagent is a liquid-stable luciferase/luciferin.

3. The method of claim 1, wherein the sponge element is detached from the guiding member (110) following introduction of the sponge element into the test swab tube.

4. The method of claim 1, further comprising:
inserting the test swab tube into an analysis device;
analyzing at least part of the contents of the test swab tube using the analysis device to produce a signal associated with a level of cleanliness of the cannula; and
outputting a representation of the signal.

5. The method of claim 4, wherein analyzing includes determining a concentration of at least one of adenosine triphosphate, protein, and ninhydrin; or further comprising determining whether a subsequent cleaning of the cannula is required based on the representation of the signal.

6. The method of claim 1, wherein the extractant comprises at least one of Triton X-100, a quaternary-based detergent, tricholoaceitc acid, and protocatechuic acid; or further comprising collecting intracellular ATP on the sponge element.

7. The method of claim 1, wherein the testing device (100) further comprises a test swab containing a liquid-stable luciferase/luciferin; or wherein the test swab tube is configured for at least partial placement within a hand held device configured to output a representation of the cleanliness of the cannula.

## Patentansprüche

1. Verfahren zur Prüfung einer Sauberkeit einer Kanüle eines medizinischen Instruments unter Verwendung einer Prüfvorrichtung (100), wobei die Prüfvorrichtung (100) ein Führungselement (110) umfasst, das ein abgerundetes erstes Ende (101) und ein zweites Ende (102) aufweist, das lösbar an ein Schwammelement gekoppelt ist, das ein getrocknetes Extraktionsmittel enthält, wobei die Schritte Folgendes umfassen:
Befeuchten des Schwammelements in mindestens einem von sterilem Wasser und ATPfreiem Wasser, um das getrocknete Extraktionsmittel zu aktivieren;
Einstecken des ersten Endes (101) des Führungselements (110) in ein proximales Ende der Kanüle;
Drücken des Führungselements (110) in die Kanüle, um das erste Ende (101) des Führungselements (110) durch die Kanüle zu führen, bis das erste Ende (101) des Führungselements (110) ein distales Ende der Kanüle verlässt;
Ziehen zumindest eines Teils des Führungselements (110) aus dem distalen Ende der Kanüle;
Berühren einer Innenfläche der Kanüle mit dem aktivierten Extraktionsmittel, um eine Zelle zu lysieren, die sich auf der Innenfläche der Kanüle befindet;
Ziehen des zweiten Endes (102) des Führungselements (110) und des Schwammelements aus dem distalen Ende der Kanüle;
Lösen des Schwammelements von dem Führungselement (110);
Einführen des Schwammelements in ein Testtupferröhrchen; und
Aussetzen des Schwammelements an ein Reagenz in dem Testtupferröhrchen.

2. Verfahren nach Anspruch 1, wobei das Reagenz eine flüssigkeitsstabile Luziferase/ein flüssigkeitsstabiles Luziferin ist.

3. Verfahren nach Anspruch 1, wobei das Schwammelement nach dem Einführen des Schwammelements in das Testtupferröhrchen von dem Führungselement (110) gelöst wird.

4. Verfahren nach Anspruch 1, ferner umfassend:
Einstecken des Testtupferröhrchens in eine Analysevorrichtung;
Analysieren zumindest eines Teils des Inhalts des Testtupferröhrchens unter Verwendung der Analysevorrichtung, um ein Signal zu erzeugen, das einem Sauberkeitsgrad der Kanüle zugeordnet ist; und
Ausgeben einer Darstellung des Signals.

5. Verfahren nach Anspruch 4, wobei das Analysieren das Bestimmen einer Konzentration von mindestens einem von Adenosintriphosphat, Protein und Ninhydrin beinhaltet; oder ferner das Bestimmen, ob eine nachfolgende Reinigung der Kanüle erforderlich ist, basierend auf der Darstellung des Signals umfasst.

6. Verfahren nach Anspruch 1, wobei das Extraktionsmittel mindestens eines von Triton X-100, einem Detergens auf Quartärbasis, Trichloressigsäure und Protocatechusäure umfasst; oder ferner umfassend das Sammeln von intrazellulärem ATP auf dem Schwammelement.

7. Verfahren nach Anspruch 1, wobei die Prüfvorrichtung (100) ferner einen Testtupfer umfasst, der eine flüssigkeitsstabile Luziferase/ein flüssigkeitsstabiles Luziferin enthält; oder wobei das Testtupferröhrchen für eine zumindest teilweise Platzierung in einer Handvorrichtung konfiguriert ist, die dazu konfiguriert ist, eine Darstellung der Sauberkeit der Kanüle auszugeben.

## Revendications

1. Procédé permettant de tester la propreté d'une canule d'un instrument médical à l'aide d'un dispositif de test (100), ledit dispositif de test (100) comprenant un élément de guidage (110) comportant une première extrémité arrondie (101) et une seconde extrémité (102) couplée de manière libérable à un élément spongieux contenant un agent d'extraction séché, les étapes comprenant :
le mouillage de l'élément spongieux dans au moins une eau parmi de l'eau stérile et de l'eau exempte d'ATP de manière à activer l'agent d'extraction séché ;
l'insertion de la première extrémité (101) de l'élément de guidage (110) dans une extrémité proximale de la canule ;
la poussée de l'élément de guidage (110) dans la canule de manière à faire passer la première extrémité (101) de l'élément de guidage (110) à travers la canule jusqu'à ce que la première extrémité (101) de l'élément de guidage (110) sorte d'une extrémité distale de la canule ;
la traction d'au moins une partie de l'élément de guidage (110) hors de l'extrémité distale de la canule ;
la mise en contact d'une surface interne de la canule avec l'agent d'extraction activé de manière à lyser une cellule située sur la surface interne de la canule ;
la traction de la seconde extrémité (102) de l'élément de guidage (110) et de l'élément spongieux hors de l'extrémité distale de la canule ;
la séparation de l'élément spongieux de l'élément de guidage (110) ;
l'introduction de l'élément spongieux dans un tube pour écouvillon d'essai ; et
l'exposition de l'élément spongieux à un réactif dans le tube pour écouvillon d'essai.

2. Procédé selon la revendication 1, ledit réactif étant une luciférase/luciférine stable aux liquides.

3. Procédé selon la revendication 1, ledit élément spongieux étant détaché de l'élément de guidage (110) après introduction de l'élément spongieux dans le tube pour écouvillon d'essai.

4. Procédé selon la revendication 1, comprenant en outre :
l'insertion du tube pour écouvillon d'essai dans un dispositif d'analyse ;
l'analyse d'au moins une partie du contenu du tube pour écouvillon d'essai à l'aide du dispositif d'analyse de manière à produire un signal associé au niveau de propreté de la canule ; et
la génération d'une représentation du signal.

5. Procédé selon la revendication 4, ladite analyse comprenant la détermination d'une concentration d'au moins un élément parmi l'adénosine triphosphate, la protéine et la ninhydrine ; ou comprenant en outre la détermination pour savoir si un nettoyage ultérieur de la canule est nécessaire sur la base de la représentation du signal.

6. Procédé selon la revendication 1, ledit agent d'extraction comprenant au moins un composé parmi le Triton X-100, un détergent à base quaternaire, l'acide trichloroacétique et l'acide cobalatéchuique ; ou comprenant en outre la collecte d'ATP intracellulaire sur l'élément spongieux.

7. Procédé selon la revendication 1, ledit dispositif de test (100) comprenant en outre un écouvillon d'essai contenant une luciférase-luciférine stable aux liquides ; ou ledit tube pour écouvillon d'essai étant conçu pour le placement au moins partiel à l'intérieur d'un dispositif portatif conçu pour générer une représentation de la propreté de la canule.
